Europäisches Patentamt

(19) European Patent Office

Office européen des brevets

(11) Numéro de publication : **0 238 586 B1**

(12) **FASCICULE DE BREVET EUROPEEN**

(45) Date de publication du fascicule du brevet :
05.06.91 Bulletin 91/23

(51) Int. Cl.⁵ : **A61B 5/103**

(21) Numéro de dépôt : 86905842.0

(22) Date de dépôt : 24.09.86

(86) Numéro de dépôt international :
**PCT/FR86/00325**

(87) Numéro de publication internationale :
**WO 87/01923 09.04.87 Gazette 87/08**

(54) **APPAREIL DE DETECTION ET DE CORRECTION D'ANOMALIES D'EQUILIBRE DU CORPS HUMAIN.**

(30) Priorité : 25.09.85 FR 8514196

(43) Date de publication de la demande :
30.09.87 Bulletin 87/40

(45) Mention de la délivrance du brevet :
05.06.91 Bulletin 91/23

(84) Etats contractants désignés :
AT BE CH DE FR GB IT LI LU NL SE

(56) Documents cités :
FR-A-20 947 00
FR-A-24 432 35
FR-A-25 429 91
US-A-23 747 30
US-A-38 261 45

(73) Titulaire : WALTHERT, Nicole, Simone
6, rue de Jargeau
F-45000 Orléans (FR)

(72) Inventeur : WALTHERT, Nicole, Simone
6, rue de Jargeau
F-45000 Orléans (FR)

(74) Mandataire : Lepeudry-Gautherat, Thérèse et al
ARMENGAUD JEUNE CABINET LEPEUDRY 6,
rue du Faubourg Saint Honoré
F-75008 Paris (FR)

## Description

L'invention a pour objet un appareil de détection et de correction d'anomalies d'équilibre du corps humain, basé sur l'interprétation de la répartition du poids du corps sur les points d'appui des pieds.

On sait que le centre de gravité du corps humain se situe au niveau de la troisième vertèbre lombaire qui constitue un des points les plus importants de la colonne vertébrale pour le maintien de l'équilibre, en même temps qu'un des plus vulnérables car elle supporte tout le poids au-dessus d'elle tandis que toute la partie du corps au-dessous d'elle lui est suspendue. Cette troisième vertèbre lombaire est le siège des lésions les plus courantes de la colonne, car dans tous les changements de posture dus à la mobilité et à la locomotion du corps humain, le centre de gravité tend à maintenir une ligne verticale allant du sommet de la tête au centre de la voûte plantaire, c'est-à-dire que tous les mouvements du corps passent par cette vertèbre. De ce fait et par ce jeu, la moindre lésion osseuse ou musculaire se traduira par une recherche de compensation dans le sens d'une ligne verticale normale entre les pieds et la tête qui se traduira par un déséquilibre et une mauvaise répartition des charges sur la voûte plantaire.

On sait que le poids du corps en position debout et immobile doit être normalement réparti sur trois points d'appui de la voûte plantaire.

La voûte plantaire ne réalise par un triangle équilatéral, mais possède trois arches et trois points d'appui qui réalisent une structure comparable. Les points d'appui sont compris dans la zone de contact avec le sol qui reçoit l'empreinte plantaire et ils correspondent à la tête du premier métatarsien, à la tête du cinquième métatarsien et aux tubérosités postérieures du calcaneum. Chaque point d'appui est commun aux deux arches contiguës. L'arche interne (calcaneum-tête du premier métatarsien) a pour clé de voûte le scaphoïde, l'arche externe (calcaneum-tête du cinquième métatarsien) a pour clé de voûte la grande apophyse du calcaneum. L'arche antérieure (tête du premier métatarsien, tête du cinquième métatarsien) a pour clé de voûte la tête du deuxième métatarsien. Le poids du corps se répartit par moitié sur l'avant-pied et l'arrière-pied, à l'aplomb du prolongement du bord antérieur de la jambe, ce qui correspond aux clés de voûte des arches interne et externe, c'est-à-dire au centre du cou de pied.

La répartition des pressions d'appui au sol est très importante pour atteindre l'équilibre. En effet, ce sont les pressions d'appui perçues par la plante des pieds qui informent le système nerveux dans son ensemble, oreille interne et cervelet compris. Par voie réflexe, tous les muscles de la verticalité, de lutte contre la pesanteur, se contractent et l'équilibre est atteint.

Dès qu'il y a donc un défaut d'appui au sol, donc un défaut d'information sensorielle plantaire, cela entraîne un défaut de stimulation nerveuse et de réponse musculaire pour aboutir à un déséquilibre ou un équilibre instable. Le centre de gravité étant déplacé, des efforts de compensation sont exercés par le corps entraînant des déformations, des douleurs, voire même des vertiges et une atteinte de la coordination.

Pour remédier à ces perturbations, il apparaît donc nécessaire d'une part que la personne prenne conscience et apprécie l'anomalie d'équilibre de son corps, et d'autre part soit en mesure de corriger cette anomalie en rectifiant progressivement sa position jusqu'à aboutir à une répartition aussi bonne que possible de son poids.

Il existe des appareils qui permettent à l'utilisateur de visualiser la répartition de son poids sur chacune de ses jambes et qui comprennent essentiellement deux surfaces mobiles d'appui, indépendantes et pourvues d'un dispositif positif de liaison du genre des tringleries existant sur les balances, qui traduit sur un cadran la différence de poids appliquée sur chacune de ces surfaces. On adjoint habituellement à ces appareils des dispositifs annexes, comme des miroirs quadrillés grâce auxquels la personne peut avoir l'image de son corps et rectifier de lui-même la dissymétrie de sa position.

On connaît aussi une réalisation telle que décrite dans le FR-A 2 443 235, dans laquelle deux étages de plateaux superposés reposent sur le sol avec interposition d'amortisseurs entre chaque étage, l'étage supérieur étant constitué de deux plateaux indépendants sur lesquels les pieds sont en appui, chaque plateau étant associé à un indicateur d'inclinaison.

D'autres systèmes permettent de déceler de mauvaises répartitions de poids par l'examen d'empreintes des pieds plus ou moins marquées sur un support.

Ces dispositifs présentent de nombreux inconvénients car ils ne donnent que des indications grossières du déséquilibre du corps et la compensation recherchée ne peut alors consister qu'à rectifier la verticalité générale du corps ce qui est loin d'être suffisant.

Par ailleurs, les systèmes connus à plusieurs plateaux sont compliqués à réaliser, difficiles à régler, et d'un maniement très délicat par l'utilisateur.

L'invention apporte une solution à ce problème en ce qu'elle propose un appareil qui permet de déceler non seulement une mauvaise répartition du poids d'une personne sur ses deux pieds, mais aussi un déséquilibre de répartition du poids sur la voûte plantaire, appareil qui est en outre extrêmement précis, très simple à fabriquer et à utiliser, ce qui le rend particulièrement apte à susciter chez de nombreuses personnes des réactions de correction efficaces.

L'appareil selon l'invention est définie par les caractéristiques de la revendication 1.

D'autres caractéristiques particulières et avantages de l'invention ressortiront de la description qui va suivre de formes de réalisations données à titre d'exemples, et en référence aux dessins annexés qui représentent :

  – Figure 1, une vue en perspective de l'appareil ;

  – Figure 2, une vue en plan du plateau-support montrant un appui des deux pieds ;

  – Figure 3, une vue en perspective d'une variante de réalisation du plateau supérieur ;

  – Figure 4, une vue en plan d'une variante de réalisation du plateau-support ;

  – Figures 5 et 8 deux vues en perspective d'une variante de réalisation de l'appareil ;

  – Figures 6 et 7 deux vues schématiques en perspective montrant des variantes de montage du niveau.

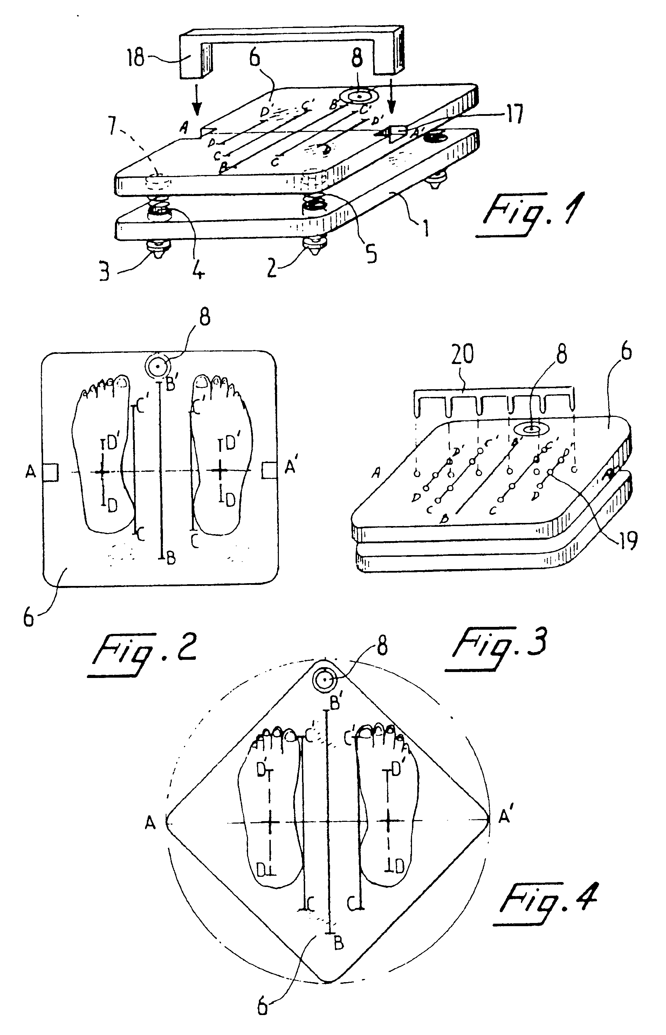

L'appareil représenté à la figure 1 comporte essentiellement un socle 1 en appui sur le sol par l'intermédiaire de quatre supports réglables en hauteur par une molette 3. Le socle est muni à ses quatre coins, au droit des pieds, de quatre excavations 4 servant de logement à des ressorts 5. Ces ressorts avantageusement en acier sont de hauteur égale, de tare égale et d'une résistance suffisante à l'écrasement pour éviter que le plateau ne puisse venir en butée contre le socle 1 quand il supporte sa charge. Un plateau supérieur 6 de dimension analogue à celle du socle repose par ses quatre coins sur les ressorts 5. Il est également muni d'excavations 7 dans lesquelles viennent se positionner lesdits ressorts. A la partie supérieure du plateau est enchâssé un niveau sphérique 8 éventuellement recouvert d'une loupe grossissante. Le plateau 6 est réalisé en matériau rigide et non déformable tel qu'un contre-plaqué latté, une matière plastique ou une tôle assez épaisse.

L'appareil fait apparaître sur son plateau supérieur une ligne transversale AA' et une ligne axiale BB' selon l'axe des pieds à l'extrémité de laquelle se trouve le niveau 8, les deux lignes étant les médianes du carré constitué par le plateau 6. D'autres lignes CC' et DD' parallèles à la ligne BB' sont disposées symétriquement de part et d'autre de celle-ci, à intervalles réguliers, les lignes extérieures DD' étant sensiblement à égale distance du bord du plateau et de la ligne axiale BB'. L'utilisateur peut ainsi placer les bords internes de chaque pied le long des lignes CC' ou DD' pour que ses appuis du pied droit et du pied gauche soient répartis symétriquement par rapport à la ligne axiale BB' comme on le voit plus précisément à la figure 2.

Afin de parfaire la position des pieds sur l'appareil, sur la ligne transversale AA', véritable ligne de partage du poids du corps (moitié vers l'avant, moitié vers l'arrière), des encoches verticales 17, situées à chaque extrémité de cette ligne permettront le passage d'un système de calage des pieds constitué de préférence de matériau rigide et ayant la forme de la moitié d'un cadre 18 dont les bords libres glissent dans les encoches ou rainures 17. Les bords libres sont suffisamment hauts pour permettre de caler les pieds, même chaussés de talons hauts.

Selon une variante de réalisation non représentée, le cadre 18 peut être remplacé par un matériau élastique, de forme semblable à celle d'un bandeau. Ce bandeau passerait autour de l'appareil, dessous et dessus, serait retenu dans les encoches 17, de façon à être parfaitement superposé à la ligne transversale AA'. Le sujet glisserait ses pieds sous le bandeau élastique jusqu'à ce que le bord antérieur de la jambe soit atteint.

Pour positionner correctement les pieds à l'aide du cadre rigide 18 on place les pieds le long des lignes CC' ou DD', comme indiqué précédemment et on les engage sous le cadre jusqu'à ce que le bord antérieur de la jambe vienne en butée contre la partie horizontale du cadre. Le centre du cou de pied correspondant aux clés de voûte des arches internes et externes du point d'appui du corps, se trouve donc centré sur les points de rencontre de la ligne transversale AA' avec les lignes axiales CC' et DD' soulignées par des + visibles à la figure 2. Il est recommandé de marquer à l'aide d'un crayon, l'empreinte de chaque pied sur le plateau supérieur 6 afin d'éviter le calage à chaque exercice. Il est évident que les systèmes de calages seront retirés après le bon positionnement des pieds sur l'appareil.

Enfin, et selon une variante de réalisation illustrée à la figure 3, pour un placement des pieds parfait dans le sens longitudinal et dans le sens transversal, les lignes CC' et DD', parallèles et équidistantes de la grande ligne médiane BB', pourront être percées de petits trous 19, dans lesquels viendront se placer les six dents d'un peigne 20, le peigne dépassant la surface du plateau et servant de guide ou de calage au bord interne de chaque pied. En ce qui concerne le calage transversal, ce même système de trous dans la ligne transversale permettant l'utilisation du peigne est possible. Les trous pourront traverser dans tous les cas tout le plateau supérieur 6 dans son épaisseur. En cas d'essai de l'appareil avec port de chaussures, on dégagera le peigne en tirant vers le haut jusqu'à ce qu'il vienne en contact de la partie inférieure du bord antérieur de la jambe. Dans tous les cas, les systèmes de calage seront enlevés après placement des pieds et avant utilisation de l'appareil.

L'appareil est utilisé pour le diagnostic et la correction du déséquilibre debout, sans bouger, mais aussi de l'équilibre en arrêt de mouvement, comme par exemple, l'équilibre accroupi. Si le pied doit rigoureusement rester appuyé sur le plateau supérieur par ses trois points d'appui, et ne pas se déplacer malgré les changements de forme des arches qui ont tendance à s'écraser normalement, la jambe, par contre, change d'orientation et tout système de calage transversal laissé en place serait un frein à l'exécution de la position accroupie. L'invention repose sur un parfait positionnement des pieds sur l'appareil. En effet, on sait que l'équilibre vertical de l'homme sur deux pieds ou sur un pied, correspond à la juste répartition de son poids sur les trois points d'appui de la voûte plantaire. Avec cette invention, les pieds correctement placés, le niveau préalablement réglé à l'aide des supports réglables, on est sûr que lorsque la bulle du niveau 8 reste centrée, quand on est en position debout sur l'appareil ou accroupie, que les pressions de la voûte plantaire sur le sol matérialisé ici par le plateau supérieur de l'appareil, sont correctement réparties.

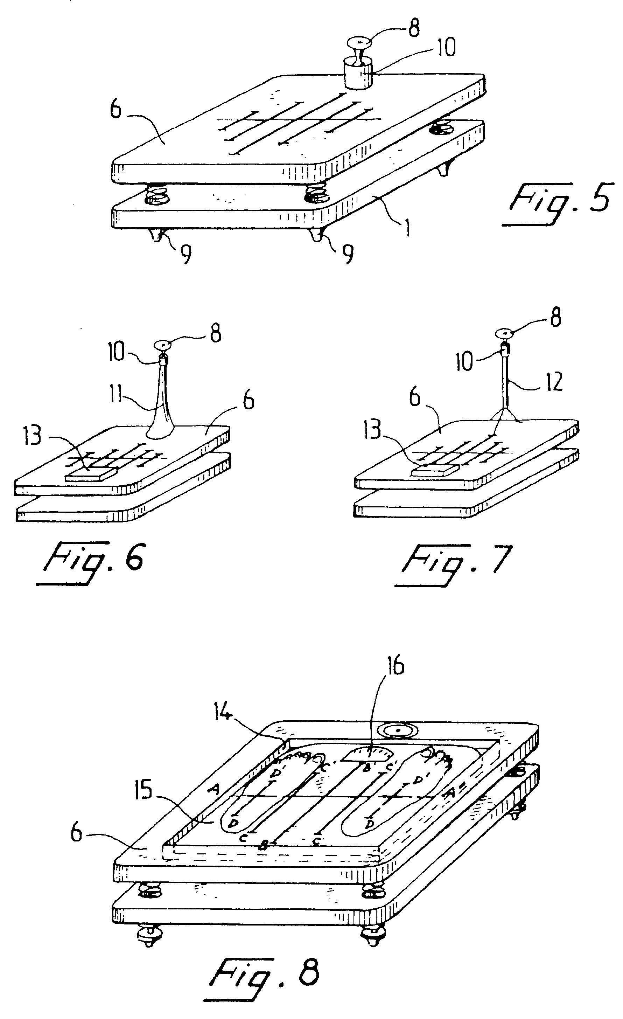

La figure 4 illustre une variante de réalisation dans laquelle le plateau est carré, mais le niveau est localisé dans un angle et les lignes CC' et DD' orientées comme représenté de part et d'autre de la ligne axiale BB'. La forme extérieure du plateau 6 peut aussi être circulaire comme représenté en pointillé.

D'autres variantes de réalisation sont illustrées par les figures 5 à 8.

A la figure 5 le socle A est en appui sur le sol par l'intermédiaire de pieds fixes 9. Pour compenser la planéité du sol, le niveau 8 est alors monté sur une rotule de réglage 10 qui permet de centrer la bulle et par conséquent de compenser l'irrégularité du sol avant d'utiliser l'appareil.

Ce montage à rotule permettrait d'utiliser également un plateau supérieur incliné par rapport au sol.

Dans les réalisations montrées aux figures 6 et 7, le niveau 8 est fixé sur une colonne 11 ou sur une tige 12 montée sur trépied et réglable en hauteur, toutes deux solidaires du plateau 6. Chacune porte également une rotule de réglage 10. Cette disposition rapproche le niveau de l'oeil de l'utilisateur. Un contrepoids 13 est fixé par ailleurs sur le plateau pour compenser le poids de la colonne ou de la tige.

Dans la représentation de la figure 8, le plateau 6 est muni d'un évidement central 14 qui sert de logement à un pèse-personne 15 muni de son indicateur de pesée 16. Le pèse-personne est centré de telle façon que l'utilisateur puisse placer ses pieds sur celui-ci dans l'alignement des lignes axiales CC' DD' et AA'.

L'appareil ainsi décrit dans ses multiples variantes est utilisé de la façon suivante, après que la bulle du plateau 6 ait été centrée à l'aide des supports réglables.

Après avoir positionné les pieds le long des lignes CC' ou DD' puis par rapport au système de calage à cadre ou à peigne, on examine la position de la bulle.

Si le poids est réparti normalement sur les points d'appui des pieds, le centre de gravité du corps est à sa bonne place et la bulle du niveau 8 est parfaitement centrée.

Toute excentration de cette bulle correspond à un déséquilibre du corps et une mauvaise répartition du poids au niveau de son pied. L'utilisateur le perçoit visuellement et peut assurer lui-même par action musculaire la correction de son anomalie d'équilibre en accentuant la pression d'un pied ou des deux vers l'avant ou l'arrière pour rapprocher progressivement la bulle de son point central. La position de la bulle avant correction renseigne l'utilisateur sur l'orientation de la compensation qu'il doit effectuer. L'appareil permet ainsi à l'utilisateur d'assurer la correction aussitôt après ce diagnostic du déséquilibre, car il a eut conscience de sa perception plantaire qui est un facteur essentiel du maintien de l'équilibre, ce qui a entraîné chez lui une réaction ostéoarticulaire et musculaire. La répétition de ces mouvements de recherche du centrage de la bulle conduisent l'utilisateur à progressivement éliminer les insuffisances de certains muscles en les obligeant à travailler pour parvenir à l'équilibre du corps.

Ainsi, cette invention permet d'étudier les transferts de poids au niveau des pieds sur des sols de différentes inclinaisons et de faire des diagnostics quant aux défauts d'appui au niveau de chaque point d'appui de la voûte plantaire. La possibilité qu'offre l'invention est importante dans la pratique de différents sports où les transferts d'appui sont nécessaires et demandent une fine coordination : par exemple skis, planche à voile, surf, golf... etc.

Les exercices, qu'ils soient sur un sol plat, bulle du niveau centrée sur le plateau parallèle au sol, ou bulle du niveau centrée sur un sol incliné, en surélevant un ou deux pieds de l'appareil, placent toujours le centre de gravité du corps à sa place. Les exercices peuvent être des accroupissements où tout le corps entre en jeu dans la recherche de l'équilibre. Mais ils peuvent être aussi des exercices qui laissent fixe la partie supérieure du corps à partir du centre de gravité et mobilisent la partie inférieure du corps ou inversement.

Après utilisation, l'usager ressent un appui correct, confortable, ce que l'on appelle l'aplomb sur le sol. Il se tient mieux verticalement, marche mieux et entretient sa musculature stabilisatrice dans tous ses mouvements de façon correcte, ceci ; à partir des informations acquises pendant l'utilisation de l'appareil. Un entretien quotidien et l'exécution de différents exercices de courte durée sur l'appareil suffit à corriger la posture juste, à corriger les déformations en cours, à redonner un tonus correct à tous les muscles de l'équilible, donc à redonner équilibre et souplesse, coordination... au corps.

Ainsi, cette invention permet d'étudier les déséquilibres dans de nombreuses conditions et de déceler les imperfections d'appui en même temps que les réponses musculaires insatisfaisantes et de les corriger. Avec cette invention, toutes les chaînes musculaires de la posture peuvent être étudiées. Le système nerveux étant le lien entre l'appui au sol et la réponse musculaire de l'équilibre, on comprend que cet appareil trouvera de nombreuses applications en neurologie.

L'invention à été décrite en référence à certaines formes de réalisation de l'appareil montrées aux figures 1 à 8. D'autres formes de réalisation non représentées font également partie de l'invention. De même certaines parties constitutives de l'appareil peuvent être modifiées sans sortir du cadre de l'invention.

Ainsi, le niveau utilisé pourrait être un pendule dont les déplacements seraient décelés et transmis électriquement, par exemple par des résistances ou des contacteurs électriques associés à des indicateurs lumineux.

## Revendications

1. Appareil de détection et de correction d'anomalies d'équilibre du corps humain comportant un organe indicateur de l'horizontalité d'un mécanisme mobile du genre d'un niveau à bulle monté sur au moins un organe déformable (5) et sur lequel est placé l'utilisateur, caractérisé en ce que ledit mécanisme est un unique plateau mobile (6) qui repose par l'intermédiaire d'au moins trois organes déformables (5) sur un socle fixe (1) lui-même en appui sur le sol, en ce que l'organe indicateur (8) est disposé à la partie supérieure du plateau et en ce que ledit plateau mobile porte des éléments de marquage du positionnement de l'un ou des deux pieds constitués de lignes axiales (CC', DD') parallèlement à une ligne axiale médiane (BB') du plateau, et symétriquement de part et d'autre de celle-ci, ainsi qu'un système de calage amovible (18, 20) du pied par rapport à une ligne transversale médiane (AA') de partage du poids du corps.

2. Appareil selon la revendication 1, caractérisé en ce que le système de calage amovible est constitué d'un demi-cadre rigide (18) coopérant avec des encoches (17) prévues sur le plateau (6) aux extrémités de la ligne transversale AA'.

3. Appareil selon la revendication 1, caractérisé en ce que le système de calage amovible est constitué d'un bandeau élastique retenu par des encoches (17) prévues sur le plateau (6) aux extrémités de la ligne transversale AA'.

4. Appareil selon la revendication 1, caractérisé en ce que le système de calage amovible est constitué d'un peigne (20) coopérant avec des trous (19) prévus sur les lignes axiales (CC', DD') et la ligne transversale AA'.

5. Appareil selon la revendication 1, caractérisé en ce que l'organe indicateur de l'horizontalité du plateau (6) est un pendule dont les déplacements sont décelés et transmis par des résistances électriques.

6. Appareil selon la revendication 1, caractérisé en ce que le niveau à bulle (8) est monté sur une rotule de réglage.

7. Appareil selon la revendication 1, caractérisé en ce que le plateau mobile (6) est muni d'un évidement central (14) servant de logement à un pèse-personne additionnel (15).

## Ansprüche

1. Vorrichtung zur Feststellung und Korrektur von Gleichgewichtsstörungen des menschlichen Körpers, mit einem Anzeigemittel für die horizontale Einstellung eines beweglichen Mechanismus der Art einer Libellenwaage, die auf mindestens einem deformierbaren Mittel (5) angebracht ist, wobei der Benutzer darauf plaziert wird, dadurch gekennzeichnet, daß der genannte Mechanismus eine einzige bewegliche Platte (6) ist, die über wenigstens drei deformierbare Mittel (5) auf einem festen Sockel (1) gelagert ist, der seinerseits auf dem Boden abgestützt ist, und daß das Anzeigemittel (8) am oberen Teil der Platte angeordnet ist und die genannte bewegliche Platte Elemente zur Positionsmarkierung für einen oder zwei Füße trägt, die aus axialen Linien (CC', DD') besteht, welche parallel zu einer axialen Mittellinie (BB') der Platte und beiderseits dieser Linie symmetrisch hierzu verlaufen, und daß für die Füße ein abnehmbares Verkeilungssystem (18, 20) bezüglich einer quer verlaufenden Mittellinie (AA') zur Aufteilung des Körpergewichts vorgesehen ist.

2. Vorrichtung nach Anspruch 1, dadurch gekennzeichnet, daß das lösbare Verkeilungssystem aus einem starren Element (18) nach Art eines halben Türstocks besteht, das mit Ausnehmungen (17) zusammenwirkt, die auf der Platte (6) an den Enden der Querlinie (AA') vorgesehen sind.

3. Vorrichtung nach Anspruch 1, dadurch gekennzeichnet, daß das lösbare Verkeilungssystem aus einem elastischen Band besteht, das an Ausnehmungen (17) gehalten wird, die auf der Platte (6) an den Enden der Querlinie (AA') vorgesehen sind.

4. Vorrichtung nach Anspruch 1, dadurch gekennzeichnet, daß das lösbare Verkeilungssystem aus einem Kamm (20) besteht, der mit Löchern (19) zusammenwirkt, die auf den axialen Linien (CC', DD') und der Querlinie (AA') vorgesehen sind.

5. Vorrichtung nach Anspruch 1, dadurch gekennzeichnet, daß das Anzeigemittel für die horizontale Einstellung der Platte (6) ein Pendel ist, dessen Stellungsänderungen erfaßt und über elektrische Widerstände übermittelt werden.

6. Vorrichtung nach Anspruch 1, dadurch gekennzeichnet, daß die Libellenwaage (8) auf einem Kugelgelenk zur Einstellung angebracht ist.

7. Vorrichtung nach Anspruch 1, dadurch gekennzeichnet, daß die bewegliche Platte (6) mit einer zentralen Ausnehmung (14) versehen ist, die zur Aufnahme einer zusätzlichen Personenwaage (15) vorgesehen ist.

## Claims

1. Apparatus for the detection and correction of anomalies in the equilibrium of the human body comprising a member indicating the horizontal level of a mobile mechanism of the spirit level type mounted on at least one deformable member (5) on which the user is positioned, characterized in that this mechanism is a single mobile plate (6) which rests via at least three deformable members (5) on a fixed base (1) itself bearing on the ground, in that the indicator member (8) is disposed in the upper portion of the plate and in that the mobile plate bears position marking members for one or both feet formed by axial lines (CC', DD') parallel to a median axial line (BB') of the plate and symmetrical on either side of the latter, and a detachable system (18, 20) for wedging the foot with respect to a median transverse line (AA') of body weight distribution.

2. Apparatus as claimed in claim 1, characterized in that the detachable wedging system is formed by a rigid half-frame (18) engaging with notches (17) provided on the plate (6) at the ends of the transverse line (AA').

3. Apparatus as claimed in claim 1, characterized in that the detachable wedging system is formed by an elastic strip held by notches (17) provided on the plate (6) at the ends of the transverse line (AA').

4. Apparatus as claimed in claim 1, characterized in that the detachable wedging system is formed by a comb (20) engaging with holes (19) provided on the axial lines (CC', DD') and the transverse line (AA').

5. Apparatus as claimed in claim 1, characterized in that the member indicating the horizontal nature of the plate (6) is a pendulum whose displacements are detected and transmitted by electrical resistors.

6. Apparatus as claimed in claim 1, characterized in that the spirit level (8) is mounted on an adjustment wheel.

7. Apparatus as claimed in claim 1, characterized in that mobile plate (6) is provided with a central recess (14) acting as a housing for supplementary weighing scales (15).

_Fig. 1_

_Fig. 2_

_Fig. 3_

_Fig. 4_

_Fig. 5_

_Fig. 6_

_Fig. 7_

_Fig. 8_